# FASCICULE DE BREVET EUROPEEN

(11) **EP 2 912 436 B1**
(45) Date de publication et mention de la délivrance du brevet: **21.10.2020**
(21) Numéro de dépôt: 13783910.6
(22) Date de dépôt: 28.10.2013
(51) Int. Cl.: G01N 21/47, C12M 1/34, C12M 1/22

(54) **PROCÉDÉ D'OBSERVATION D'ESPÈCES BIOLOGIQUES**
VERFAHREN ZUR BEOBACHTUNG BIOLOGISCHER SPEZIES
METHOD FOR OBSERVING BIOLOGICAL SPECIES

(30) Priorité: 29.10.2012 FR 1260301
(43) Date de publication de la demande: 02.09.2015
(73) Titulaire: Commissariat à l'Énergie Atomique et aux Énergies Alternatives, 75015 Paris (FR); Biomerieux, 69280 Marcy-l'Étoile (FR)
(72) Inventeur: DUPOY, Mathieu, F-38100 Grenoble (FR); DEBOURDEAU, Mathieu, F-74540 Cusy (FR); PINSTON, Frédéric, F-38000 Grenoble (FR)
(74) Mandataire: Gevers & Orès
(86) Numéro de dépôt international: PCT/EP2013/072530
(87) Numéro de publication internationale: WO 2014/067907

(56) Documents cités:
- EP-A2- 2 184 346
- FR-A1- 2 786 498
- FR-A1- 2 938 917
- JP-A- 5 184 349
- JP-A- 2004 012 398
- JP-A- 2010 051 200
- US-A- 3 712 746
- US-B1- 6 238 879

## Description

L'invention porte sur un procédé d'observation, et le cas échéant de détection, d'espèces biologiques sur un milieu de culture contenu dans un récipient tel qu'une boîte de Pétri.

Les micro-organismes tels que les bactéries sont généralement cultivées sur un milieu de culture (gélose) contenu dans une boîte de Pétri. Les boîtes de Pétri sont des boîtes cylindriques peu profondes, constituées d'une base et d'un couvercle, réalisées en matériau transparent tel que le verre ou une matière plastique (polystyrène). La détection des microorganismes se fait généralement par observation à l'œil nu sous un éclairage uniforme ; les colonies bactériennes apparaissent en général sous forme d'amas de matière plus ou moins bombés, qui peuvent être vus à travers les parois ou le fond de boîte de Pétri. EP2184346-A2 divulgue une méthode d'imagerie du fond d'une boîte de Pétri inversée. Un faisceau laser illumine la culture dans la boîte à un angle par rapport à la face du couvercle, pour induire une fluorescence intrinsèque dans l'échantillon. Une caméra disposée perpendiculairement au-dessus de la boîte de Pétri, capture l'image de fluorescence. L'observation à travers le couvercle est généralement rendue difficile ou impossible par la fine couche de buée, constituée par des gouttelettes diffusantes de diamètre compris entre 1 µm et 1 mm environ, qui recouvre la surface intérieure de ce dernier. Ainsi, lorsque le milieu de culture est diffusant ou absorbant (cas des géloses au sang) il est nécessaire d'ouvrir la boîte de Pétri pour observer les micro-organismes cultivés ; mais cela entraine un risque de contamination.

L'invention vise à résoudre ce problème. Plus particulièrement, elle vise à procurer un procédé permettant d'observer des espèces biologiques sur un milieu de culture contenu dans un récipient à travers une face translucide (et donc diffusante) de ce dernier, la face pouvant être rendue translucide du fait d'un dépôt de matière diffusante, en particulier de la buée, sur celle ci.

Conformément à l'invention, ce but est atteint par un procédé d'observation d'espèces biologiques sur un milieu de culture contenu dans un récipient présentant au moins une face translucide, le procédé comportant les étapes consistant à :
a) diriger un faisceau lumineux sur une portion de ladite face translucide, de façon à définir au moins une région éclairée et au moins une région non éclairée de ladite face ; et
b) acquérir une image d'une portion de la surface dudit milieu de culture éclairée par ledit faisceau lumineux, l'acquisition étant réalisée à travers ladite ou au moins une dite région non éclairée de ladite face translucide et selon un axe optique d'acquisition formant un angle non nul avec la direction de propagation dudit faisceau lumineux.

Avantageusement, les étapes a) et b) peuvent être exécutées une pluralité de fois en définissant des régions éclairées et non éclairées de ladite face translucide différentes, le procédé comportant également une étape c) de combinaison des images ainsi acquises pour former une image dite combinée.

En particulier, les étapes a) et b) peuvent être exécutées une pluralité de fois, le faisceau balayant alors la surface du milieu de culture. A chaque position successive du faisceau, on acquiert une image, lesdites images étant ensuite traitées pour former une image combinée. L'image combinée constitue alors une représentation du milieu de culture.

Par exemple, ladite image combinée peut être obtenue en associant à chaque point de la surface du milieu de culture la plus faible intensité lumineuse mesurée, en correspondance dudit point, sur lesdites images acquises.

Ladite image combinée peut également être obtenue en identifiant une région d'intérêt sur chacune des images acquises, lesdites régions d'intérêt étant ensuite associées pour former l'image combinée.

En particulier, la région d'intérêt peut comprendre la projection du faisceau lumineux sur le milieu de culture. Par exemple, la région d'intérêt peut correspondre à la projection du faisceau lumineux sur le milieu de culture. De préférence, ladite région d'intérêt ne comprend pas la projection du faisceau lumineux sur la face translucide.

A la suite des étapes a) et b) mentionnées ci-dessus, on peut extraire une zone d'intérêt de l'image, incluant la projection du faisceau lumineux sur le milieu de culture. De préférence, cette zone d'intérêt extraite ne comporte pas la projection du faisceau lumineux sur la face translucide du récipient.

Le procédé peut comprendre également une étape d) consistant à détecter lesdites espèces biologiques par discrimination, sur ladite image ou image combinée, de régions claires ou sombres. Cette détection peut être effectuée par un opérateur, ou bien de manière automatique par un ordinateur exécutant un logiciel de traitement des images. Cette détection peut s'accompagner d'un dénombrement des colonies, ainsi que de leur classement selon un critère donné, par exemple leur surface.

Avantageusement, ledit axe optique d'acquisition peut former un angle d'au moins 10°, et de préférence compris entre 30° et 60°, avec ladite face translucide et selon un axe optique d'acquisition formant un angle avec la direction de propagation dudit faisceau lumineux.

Ledit faisceau lumineux peut notamment définir, sur ladite face translucide, une région éclairée en forme de ligne.

Selon un premier mode de réalisation de l'invention, ledit axe optique d'acquisition ne coïncide pas avec la direction de réflexion spéculaire dudit faisceau lumineux par ledit milieu de culture, moyennant quoi lesdites espèces biologiques apparaissent sur ladite image comme des régions claires.

Selon un deuxième mode de réalisation de l'invention, ledit axe optique d'acquisition coïncide sensiblement avec la direction de réflexion spéculaire dudit faisceau lumineux par ledit milieu de culture, moyennant quoi lesdites espèces biologiques apparaissent sur ladite image comme des régions sombres.

Ladite face peut être rendue translucide par un dépôt de matière diffusante, en particulier de gouttelettes de buée. Plus particulièrement, ledit récipient peut être une boîte, notamment une boîte de Pétri et ladite face translucide est un couvercle de ladite boîte, dont la surface intérieure est recouverte de buée.

D'une façon plus générale, la face translucide est disposée en vis-à-vis des espèces biologiques. De préférence, la face translucide n'est pas en contact avec lesdites espèces biologiques.

D'autres caractéristiques, détails et avantages de l'invention ressortiront à la lecture de la description faite en référence aux dessins annexés donnés à titre d'exemple, dans lesquelles:
- la figure 1 montre une boîte de Pétri présentant une couche de buée sur la surface intérieure de son couvercle, illustrant le problème résolu par l'invention ;
- les figures 2A - 2C représentent schématiquement la mise en œuvre d'un procédé selon un mode de réalisation de l'invention ;
- les figures 3A - 3D illustrent le résultat technique de l'invention, appliquée à la détection de colonies bactériennes ;
- la figure 4 illustre le résultat technique de l'invention, appliquée à la détection de moisissures sur un milieu de culture dans une boîte de Pétri ;
- les figures 5A - 5C illustrent la mise en œuvre d'un procédé selon un mode de réalisation alternatif de l'invention ; et
- les figures 6A et 6B permettent de comparer une image obtenue en conditions de réflexion spéculaire (6A) et une image obtenue hors conditions de réflexion spéculaire (6B).

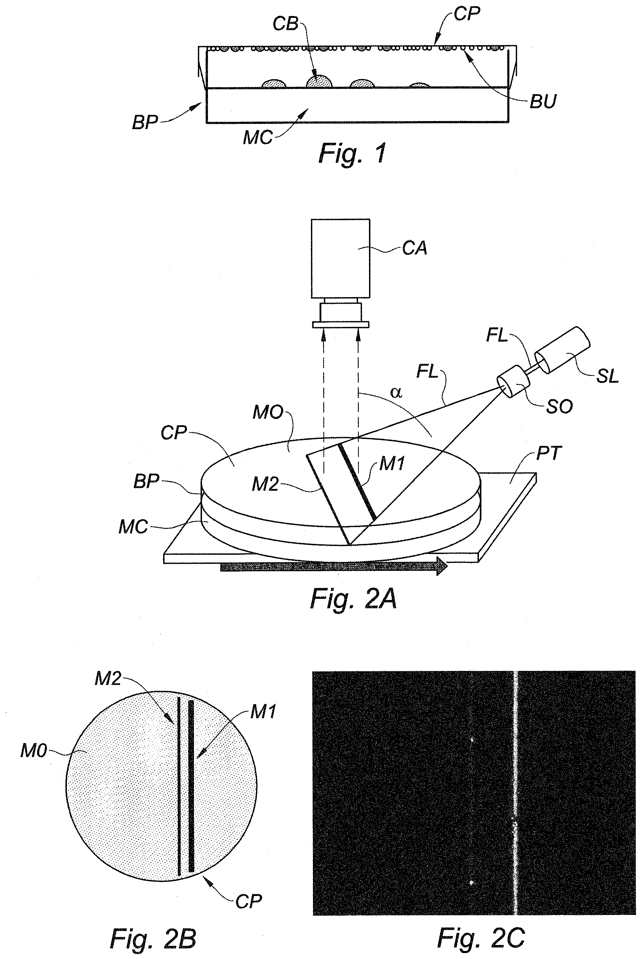

La figure 1 montre une vue en coupe d'une boîte de Pétri BP fermée de manière étanche par un couvercle CP, dont la surface intérieure est couverte d'une fine couche de buée BU ; alors que le couvercle CP est en lui-même transparent, il est rendu translucide par la diffusion provoquée par les gouttelettes d'eau constituant la couche de buée. La boîte de pétri est partiellement remplie d'un milieu de culture MC de type gélose, éventuellement opaque ou diffusante, sur lequel sont cultivées des colonies bactériennes CB. Comme expliqué plus haut, d'une part la buée BU empêche l'observation des colonies CB à travers le couvercle, d'autre part enlever le couvercle engendrerait un risque de contamination du milieu de culture ou de l'environnement extérieur à la boîte de Pétri.

Ce problème peut être résolu, conformément à l'invention, de la manière illustrée sur la figure 2A. Une source de lumière SL (par exemple un laser à une longueur d'onde de 532 nm) émet un faisceau lumineux FL qui est mis en forme par un système optique. Le faisceau mis en forme est dirigé obliquement vers le couvercle CP, de telle sorte que son intersection avec ledit couvercle définit un motif d'éclairage M1 en forme de ligne, présentant une longueur au moins dix fois supérieure à sa largeur qui est de l'ordre de 1 mm ou moins. La référence M2 indique le motif défini sur la surface du milieu de culture MC. On indique par M0 la partie du couvercle qui n'est pas éclairée (et donc extérieure à M1). Une caméra CA, orientée selon la normale à la surface du couvercle - et formant donc un angle α≠0° avec le faisceau lumineux d'éclairage - acquiert une image du couvercle CP (figure 2B).

Etant donné que la direction de propagation du faisceau FL et la direction d'observation de la caméra (c'est-à-dire son axe optique d'acquisition des images) forment un angle non nul, la caméra observe la région éclairée du milieu de culture (motif M2) à travers une partie non éclairée du couvercle (M0). Les colonies bactériennes CB apparaissent comme des points brillants superposés au motif M2, en raison de leur caractère diffusant. La figure 2C montre une image acquise par la caméra CA : on peut remarquer le motif M1 sous la forme d'une ligne brillante, le motif M2 sous la forme d'une ligne beaucoup moins brillante, et deux points plus lumineux qui correspondent à des colonies bactériennes. En effet :
- le milieu de culture réfléchit la lumière incidente de manière essentiellement spéculaire, mais cette réflexion n'est pas interceptée par la caméra ; seule la lumière diffusée par les irrégularités de sa surface, ou par la buée BU, est détectée ;
- les colonies bactériennes constituent justement des irrégularités de la surface du milieu de culture : pour cette raison, elles apparaissent plus lumineuses ;
- la couche de buée est très diffusante, ce qui explique la brillance du motif M1.

La boîte de Pétri est avantageusement montée sur une platine de translation, ce qui permet de réaliser un balayage de la surface du milieu de culture et de reconstituer une image complète mettant en évidence les colonies bactériennes. Ces dernières peuvent ensuite être détectées par un opérateur ou bien de manière automatique au moyen d'un procédé de traitement des images connu en soi, par exemple à l'aide d'un seuillage, d'un filtre passe haut, ou d'une détection de contours, exécuté par un ordinateur programmé de manière opportune et reliée à la caméra CA.

Pour obtenir l'image complète, on peut extraire, sur chaque image acquise, une région d'intérêt, qui correspond à la projection du faisceau sur le milieu de culture. La largeur d'une région d'intérêt est de préférence égale au pas de translation entre deux positions successives de la boîte de Pétri. Entre deux images successivement acquises, la région d'intérêt est décalée d'une distance correspondant au pas de déplacement de la platine. Les régions d'intérêt extraites au cours du balayage sont ensuite associées pour former l'image complète.

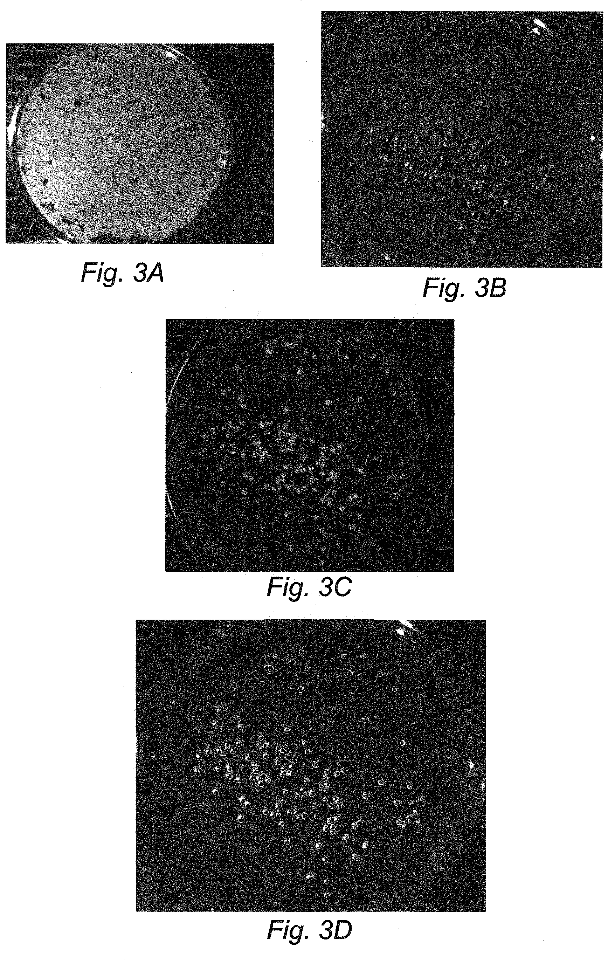

L'effet technique de l'invention est illustré par les figures 3A à 3D, qui se rapport au cas d'une culture de Escherichia Coli à 24 heures de culture sur une gélose au sang (Gélose Columbia + 5% de sang de mouton - référence Biomérieux 43041). L'angle α est égal à 42°, l'éclairement étant réalisé par une source Laser, projetant une ligne de largeur 1 mm sur le milieu de culture. La figure 3A a été acquise à travers le couvercle, éclairé de manière uniforme : on ne voit que la lumière rétrodiffusée par la buée et on ne distingue pas du tout les colonies bactériennes. Ces dernières sont en revanche clairement visibles non seulement sur la figure 3B, acquise après avoir ôté le couvercle, mais également sur la figure 3C, obtenue à travers le couvercle par la méthode décrite plus haut. La figure 3D correspond à une superposition des figures 3B et 3C, qui permet d'en vérifier leur très bonne corrélation.

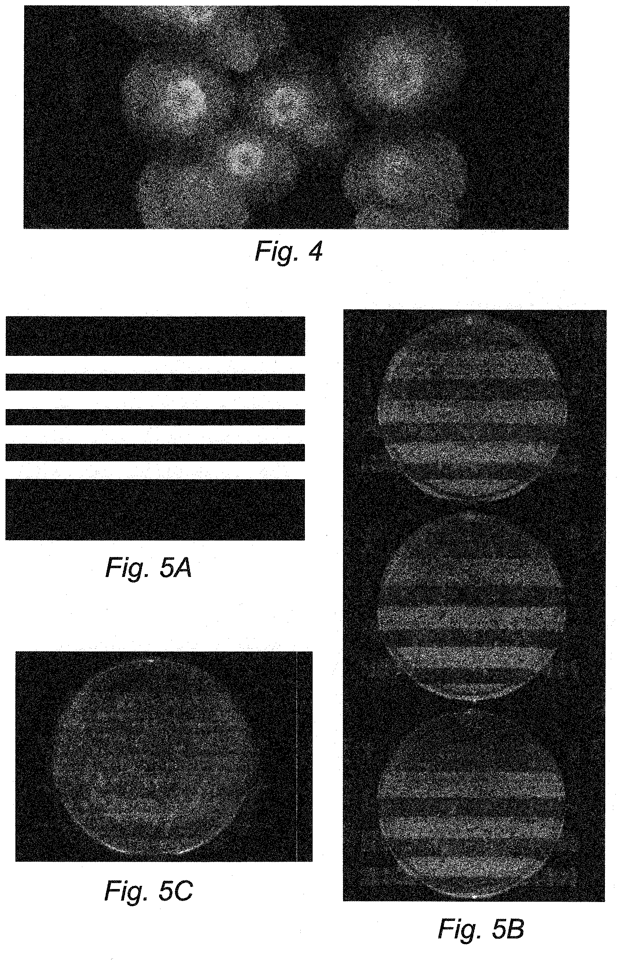

L'invention ne se limite pas à l'observation de colonies bactériennes ; à titre d'exemple la figure 4 montre une image obtenue par une méthode telle que décrite ci-dessus appliquée à l'observation d'un milieu de culture ensemencé avec une moisissure (Aspergilus Fumigatus).

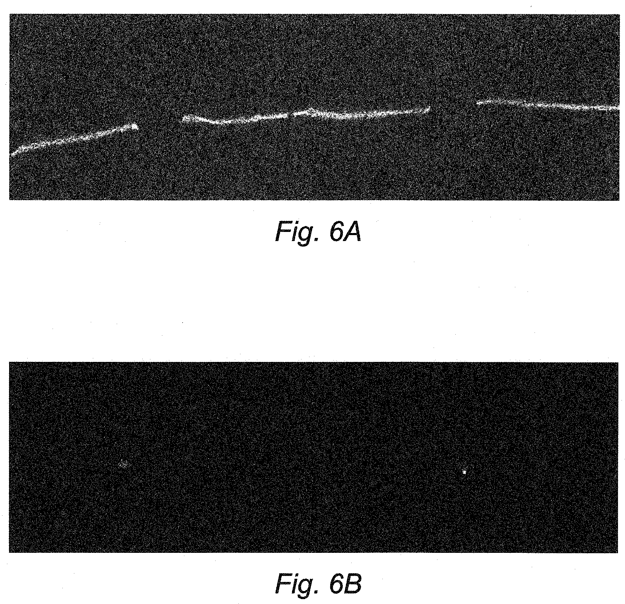

L'invention admet de nombreuses variantes :
- Le récipient peut ne pas être une boîte de Pétri, et l'observation peut ne pas se faire à travers un couvercle présentant une surface couverte de buée ; ce qui compte est qu'un milieu de culture contenu dans un récipient soit observé à travers une face dudit récipient rendue translucide (diffusante) par un dépôt de matière diffusante, par exemple de la buée ou de la graisse (traces de doigts), dans le but de détecter des espèces biologiques.
- L'éclairage peut être réalisé en lumière monochromatique ou polychromatique, voire blanche, spatialement cohérente ou incohérente.
- Plusieurs sources de lumières peuvent être utilisées : laser, lampe, diode électroluminescente, faisceau de fibres optiques, etc.
- Le faisceau lumineux peut être parallèle (collimaté), convergent ou divergeant.
- Le motif M1 ne doit pas nécessairement être en forme de ligne. Cependant, il est essentiel que le faisceau lumineux définisse au moins une région éclairée et une région non éclairée du couvercle de la boîte de Pétri (plus généralement : de la face translucide du récipient).
- Il n'est pas essentiel que le faisceau soit dirigé obliquement sur le couvercle (plus généralement : la face translucide) et que la direction d'observation soit normale audit couvercle ; l'inverse pourrait être vrai, ou alors tant la direction d'éclairage que celle d'observation pourraient être obliques. Ce qui est important est qu'une région éclairée du milieu de culture (M2) puisse être observée à travers une région non éclairée (M0) du couvercle. Pour cela, il est nécessaire que l'angle α formée par la direction d'éclairage et celle d'observation ne soit pas nul et de préférence qu'il soit supérieur ou égal à 10°.
- Un cas particulier mérite d'être signalé. Lorsque la direction d'éclairage et la direction d'observation forment un même angle avec la surface du milieu de culture, c'est-à-dire se trouvent en conditions de réflexion spéculaire, le motif M2 apparait brillant et les espèces biologiques constituent des tâches sombres. Cette situation est illustrée sur la figure 6A, où trois colonies apparaissent comme des interruptions d'une ligne d'éclairage. La figure 6B montre ces mêmes colonies qui apparaissent comme des tâches lumineuses lorsque le milieu de culture est observé hors conditions de réflexion spéculaire.

A titre d'exemple, les figures 5A à 5C illustrent un mode de réalisation alternatif de l'invention. L'éclairage est réalisé en lumière blanche et le motif M1 (figure 5A) se présente sous la forme de quatre bandes lumineuses. Trois images sont acquises en décalant une boîte de Pétri par rapport à ce motif (ce qui peut être obtenu en déplaçant la boîte, comme dans le cas de la figure 2A, ou bien le motif), comme illustré sur la figure 5B ; puis les parties sombres de ces images sont combinées entre elles pour donner une image finale, reproduite sur la figure 5C. Autrement dit, on extrait, sur chaque image, une région d'intérêt comportant la trace du faisceau lumineux sur le milieu de culture, les différentes zones d'intérêt étant combinées pour former l'image finale.

L'image 5C peut être construite pixel par pixel de la manière suivante : pour chaque point du couvercle on prend la plus faible intensité lumineuse mesurée, en correspondance dudit point, sur les différentes images acquises.

En d'autres termes, soit Iₙ (x,y) l'intensité lumineuse de l'image numéro n (n= 1 - 3 dans l'exemple des figures 5A - 5C) en fonction de la position (x,y). Alors, l'image combinée IC est formée en appliquant, à chaque point (x,y) la relation : IC(x,y) = minₙ (Iₙ(x,y)). Cela revient à masquer la trace du faisceau lumineux FL sur la face translucide du récipient, cette dernière se présentant sous la forme d'une zone d'intensité élevée.

## Revendications

1. Procédé d'observation d'espèces biologiques (CB) sur un milieu de culture (MC) contenu dans un récipient présentant au moins une face translucide (CP, BU), le procédé comportant les étapes consistant à :
a) diriger un faisceau lumineux (FL) sur une portion de ladite face translucide, de façon à définir au moins une région éclairée (M1) et au moins une région non éclairée (M0) de ladite face ; et
b) acquérir une image d'une portion (M2) de la surface dudit milieu de culture éclairée par ledit faisceau lumineux, l'acquisition étant réalisée à travers ladite ou au moins une dite région non éclairée (M0) de ladite face translucide et selon un axe optique d'acquisition formant un angle non nul (α) avec la direction de propagation dudit faisceau lumineux.

2. Procédé selon la revendication 1, dans lequel les étapes a) et b) sont exécutées une pluralité de fois en définissant des régions éclairées et non éclairées de ladite face translucide différentes, le procédé comportant également une étape c) de combinaison des images ainsi acquises pour former une image dite combinée.

3. Procédé selon la revendication 2 dans lequel ladite image combinée est obtenue en associant à chaque point de la surface du milieu de culture la plus faible intensité lumineuse mesurée, en correspondance dudit point, sur lesdites images acquises.

4. Procédé selon la revendication 2, dans lequel ladite image combinée est obtenue en définissant une région d'intérêt de chaque image acquise, lesdites régions d'intérêt étant ensuite associées pour former l'image combinée.

5. Procédé selon l'une des revendications précédentes, comprenant également une étape d) consistant à détecter lesdites espèces biologiques par discrimination, sur ladite image ou image combinée, de régions claires ou sombres.

6. Procédé selon l'une des revendications précédentes, dans lequel ledit axe optique d'acquisition forme un angle (α) d'au moins 10°, et de préférence compris entre 30° et 60°, avec ladite face translucide et selon un axe optique d'acquisition formant un angle avec la direction de propagation dudit faisceau lumineux.

7. Procédé selon l'une des revendications précédentes, dans lequel ledit faisceau lumineux définit, sur ladite face translucide, une région éclairée (M1) en forme de ligne.

8. Procédé selon l'une des revendications précédentes, dans lequel ledit axe optique d'acquisition ne coïncide pas avec la direction de réflexion spéculaire dudit faisceau lumineux par ledit milieu de culture, moyennant quoi lesdites espèces biologiques apparaissent sur ladite image comme des régions claires.

9. Procédé selon l'une des revendications 1 à 7, dans lequel ledit axe optique d'acquisition coïncide sensiblement avec la direction de réflexion spéculaire dudit faisceau lumineux par ledit milieu de culture, moyennant quoi lesdites espèces biologiques apparaissent sur ladite image comme des régions sombres.

10. Procédé selon l'une des revendications précédentes, dans lequel ladite face est rendue translucide par un dépôt de matière diffusante, en particulier de gouttelettes de buée (BU).

11. Procédé selon la revendication 10 dans lequel ledit récipient est une boîte de Pétri (BP) et ladite face translucide est un couvercle (CP) de ladite boîte, dont la surface intérieure est recouverte de buée (BU).

## Patentansprüche

1. Verfahren zum Beobachten biologischer Spezies (CB) auf einem Nährboden (MC) in einem Behälter, der mindestens eine durchsichtige Fläche (CP, BU) aufweist, wobei das Verfahren die folgenden Schritte umfasst:
a) Richten eines Lichtbündels (FL) auf einen Teil der durchsichtigen Fläche, um mindestens einen beleuchteten Bereich (M1) und mindestens einen nicht beleuchteten Bereich (M0) der Fläche zu definieren; und
b) Aufnahme eines Bildes eines Teils (M2) der Oberfläche des vom Lichtbündel beleuchteten Nährbodens, wobei die Aufnahme durch den oder mindestens einen nicht beleuchteten Bereich (M0) der durchsichtigen Fläche und gemäß einer optischen Aufnahmeachse erfolgt, die einen Winkel von ungleich Null (a) zur Ausbreitungsrichtung des Lichtbündels bildet.

2. Verfahren nach Anspruch 1, wobei die Schritte a) und b) eine Vielzahl von Malen ausgeführt werden und dabei unterschiedliche beleuchtete und nicht beleuchtete Bereiche der durchsichtigen Fläche definieren, wobei das Verfahren ebenfalls einen Kombinierungsschritt c) der so aufgenommenen Bilder umfasst, um ein als kombiniertes bezeichnetes Bild zu bilden.

3. Verfahren nach Anspruch 2, wobei das kombinierte Bild erhalten wird, indem an jedem Punkt der Oberfläche des Nährbodens die schwächste gemessene Lichtstärke, die diesem Punkt entspricht, mit den aufgenommenen Bildern verbunden wird.

4. Verfahren nach Anspruch 2, wobei das kombinierte Bild erhalten wird, indem ein Bereich von Interesse jedes aufgenommenen Bildes definiert wird, wobei die Bereiche von Interesse anschließend verbunden werden, um das kombinierte Bild zu bilden.

5. Verfahren nach einem der vorstehenden Ansprüche, das ebenfalls einen Schritt d) umfasst, der darin besteht, die biologischen Spezies durch Diskriminierung, auf dem Bild oder dem kombinierten Bild, in helle oder dunkle Bereiche zu entdecken.

6. Verfahren nach einem der vorstehenden Ansprüche, wobei die optische Aufnahmeachse einen Winkel (a) von mindestens 10°, und vorzugsweise zwischen 30 ° und 60 °, mit der durchsichtigen Fläche und gemäß einer optischen Aufnahmeachse bildet, die einen Winkel mit der Ausbreitungsrichtung des Lichtbündels bildet.

7. Verfahren nach einem der vorstehenden Ansprüche, wobei das Lichtbündel auf der durchsichtigen Fläche einen beleuchteten Bereich (M1) in Form einer Linie definiert.

8. Verfahren nach einem der vorstehenden Ansprüche, wobei die optische Aufnahmeachse nicht mit der spiegelnden Reflexionsrichtung des Lichtbündels durch den Nährboden zusammenfällt, wodurch die biologischen Spezies auf dem Bild als helle Bereiche erscheinen.

9. Verfahren nach einem der Ansprüche 1 bis 7, wobei die optische Aufnahmeachse im Wesentlichen mit der spiegelnden Reflexionsrichtung des Lichtbündels durch den Nährboden zusammenfällt, wodurch die biologischen Spezies auf dem Bild als dunkle Bereiche erscheinen.

10. Verfahren nach einem der vorstehenden Ansprüche, wobei die Fläche durch Auflage eines Diffusionsmaterials durchsichtig gemacht wird, insbesondere Kondensierungströpfchen (BU).

11. Verfahren nach Anspruch 10, wobei der Behälter eine Petrischale (BP) ist und die durchsichtige Fläche ein Deckel (CP) der Schale ist, dessen Innenfläche mit Kondensierung (BU) bedeckt ist.

## Claims

1. Method for observing biological species (CB) on a culture medium (MC) contained in a container having at least one translucent face (CP, BU), the method comprising the steps consisting of:
a) directing a light beam (FL) onto one portion of said translucent face, so as to define at least one illuminated region (M1) and at least one non-illuminated region (M0) of said face; and
b) acquiring an image of a portion (M2) of the surface of said culture medium illuminated by said light beam, the acquisition being carried out through said or at least one said non-illuminated region (M0) of said translucent face and along an optical acquisition axis forming a non-zero angle (α) with the direction of propagation of said light beam.

2. Method according to claim 1, wherein the steps a) and b) are performed a plurality of times by defining different illuminated and non-illuminated regions of said translucent face, the method also comprising a step c) of combining the images thus acquired to form a so-called combined image.

3. Method according to claim 2, wherein said combined image is obtained by associating, with each point of the surface of the culture medium, the weakest measured luminous intensity, corresponding to said point, on said acquired images.

4. Method according to claim 2, wherein said combined image is obtained by defining a region of interest of each acquired image, said regions of interest then being associated to form the combined image.

5. Method according to one of the preceding claims, also comprising a step d) consisting of detecting said biological species by discrimination, on said image or combined image, of light or dark regions.

6. Method according to one of the preceding claims, wherein said optical acquisition axis forms an angle (α) of at least 10°, and preferably between 30° and 60°, with said translucent face and along an optical acquisition axis forming an angle with the direction of propagation of said light beam.

7. Method according to one of the preceding claims, wherein said light beam defines, on said translucent face, an illuminated region (M1) in the shape of a line.

8. Method according to one of the preceding claims, wherein said optical acquisition axis does not coincide with the direction of specular reflection of said light beam by said culture medium, as a result of which said biological species appear on said image as light regions.

9. Method according to one of claims 1 to 7, wherein said optical acquisition axis substantially coincides with the direction of specular reflection of said light beam by said culture medium, as a result of which said biological species appear on said image as dark regions.

10. Method according to one of the preceding claims, wherein said face is rendered translucent by depositing a diffuser material, in particular condensation droplets (BU).

11. Method according to claim 10, wherein said container is a Petri dish (BP) and said translucent face is a lid (CP) of said dish, the inner surface of which is covered with condensation (BU).
